# DEMANDE DE BREVET EUROPEEN

(11) **EP 1 562 026 A2**
(43) Date de publication de la demande: **10.08.2005**
(21) Numéro de dépôt: 05300065.9
(22) Date de dépôt: 27.01.2005
(51) Int. Cl.: G01F 15/18

(54) **Elément de raccordement réducteur de pression, notamment pour débitmètre médical**

(30) Priorité: 04.02.2004 FR 0450203
(71) Demandeur: TAEMA, F-92182 Antony Cédex (FR)
(72) Inventeur: Bleys, Christian, 77000 Livry s/Seine (FR); Collado, Pedro, 77330 Ozoir la Ferrière (FR)
(74) Mandataire: Pittis, Olivier

(57) **Abrégé**

L'invention porte sur un élément (100) de raccordement destiné à raccorder une prise de distribution de fluide basse pression, en particulier de gaz médical, à un débitmètre (200) apte à fournir ledit fluide sous un débit donné, comprenant un corps principal comportant un raccord (110) d'entrée de fluide apte à raccorder l'élément (100) de raccordement à ladite prise de distribution ; un raccord (120) de sortie de fluide apte à raccorder l'élément (100) de raccordement audit débitmètre (200) ; un passage interne de fluide reliant le raccord (110) d'entrée au raccord (120) de sortie de fluide ; et des moyens (131,134) de réduction de la pression du fluide agencés sur ledit passage interne. Application à la distribution de fluide médical, en particulier de gaz médical.

## Description

La présente invention concerne un élément de raccordement destiné à raccorder une prise de distribution de fluide basse pression à un débitmètre apte à fournir ledit fluide sous un débit donné.

On connaît des débitmètres à application médicale, généralement des débitmètres à bille, encore appelés rotamètres, qui sont raccordés par exemple à des prises murales de distribution de fluide basse pression dans une salle ou chambre d'hôpital et qui sont destinés à délivrer, pour l'administrer à un patient, un fluide médical sous un débit réglable en continu qui doit être connu avec précision, et ceci indépendamment, dans une certaine mesure, de la basse pression amont du fluide, typiquement de quelques bars.

Actuellement, la basse pression amont au niveau des prises murales de distribution n'est pas déterminée de manière uniforme, mais est soumise à des normes ou à des réglementations spécifiques, nationales notamment. Il en résulte que les conditions de fonctionnement des débitmètres ne sont pas aujourd'hui établies de façon définitive et restent toujours soumises aux recommandations des organismes de normalisation et/ou aux décisions nationales.

Par conséquent, en cas de changements imposés concernant la pression nominale de distribution disponible en amont des débitmètres installés sur les prises de distribution murales, on doit impérativement remplacer les débitmètres existants puisqu'ils ne peuvent plus fonctionner convenablement dans les conditions de précision requise par d'autres débitmètres adaptés aux nouvelles spécifications relatives à la pression de distribution. Le remplacement, à chaque modification de norme ou de réglementation, de tout un parc de débitmètres installés se traduit bien évidemment par un coût élevé que doivent assumer les institutions hospitalières et médicales.

Le document GB-A-606,623 a proposé un élément de raccordement avec moyens internes de réduction de la pression d'un fluide sous pression et débit variable comprenant un corps principal comportant un raccord d'entrée et un raccord de sortie de fluide qui sont aptes à raccorder l'élément de raccordement sur une ligne de transport de pétrole de manière à contrôler la pression du fluide qui y circule via un passage interne de fluide reliant le raccord d'entrée au raccord de sortie de fluide.

Par ailleurs, le document US-A-3,352,147 décrit l'incorporation d'une une vanne de réduction de pression traversée par un gaz sous pression dans un système d'acheminement de gaz comportant notamment un appareil de mesure de pression du gaz.

Toutefois, ces solutions présentent l'inconvénient de réduire la pression de façon proportionnelle à la pression d'entrée du système sans possibilité d'obtenir une pression détendue fixe et constante quelle que soit la variation de pression amont.

En outre, le document EP-A-439992 propose un dispositif de mesure du flux de fluide réfrigérant destiné à un système de refroidissement comportant un passage de fluide interne avec entrée et sortie de fluide. Le passage interne comporte un piston de diamètre croissant avec ressort périphérique, ledit piston étant mobile en translation au travers d'un orifice calibré aménagé sur le passage du fluide de manière à pouvoir obturer plus ou moins l'orifice calibré, en fonction de la position du piston, et donc ajuster ainsi la quantité de fluide traversant l'orifice calibré. Il s'agit donc d'une régulation en débit et non pas en pression et cette solution n'est pas adaptée à une régulation en pression du fluide traversant le dispositif.

Aussi, le problème qui se pose est de proposer un élément de raccordement amélioré, ne présentant pas les inconvénients des dispositifs de l'art antérieur, et qui permette d'éviter le remplacement systématique des débitmètres, lorsque les normes ou la réglementation concernant la pression de distribution sont modifiées, en particulier le passage des pressions actuelles de l'ordre de 3,5 bar à une pression d'au moins 4,5 bar, voire de 5,5 bar, en amont sans modifier la pression détendue alimentant le débitmètre , et donc de limiter le coût de l'opération.

La solution proposée par la présente invention est un élément de raccordement destiné à raccorder une prise de distribution de fluide basse pression à un débitmètre apte à fournir ledit fluide sous un débit donné, comprenant un corps principal comportant :
- un raccord d'entrée de fluide apte à raccorder l'élément de raccordement à ladite prise de distribution,
- un raccord de sortie de fluide apte à raccorder l'élément de raccordement audit débitmètre,
- un passage interne de fluide reliant le raccord d'entrée au raccord de sortie de fluide, et
- des moyens de réduction de la pression du fluide agencés sur ledit passage interne,
caractérisé en ce que lesdits moyens de réduction de pression comprennent, placés entre le raccord d'entrée et le raccord de sortie, un piston soumis à l'action combinée d'une force de pression liée à la pression dans le débitmètre et à une force de rappel exercée par un moyen de rappel élastique.

En effet, la présence combinée de ce piston et du moyen de rappel élastique permet de garantir une pression de détente stable quelle que soit la variation de pression en amont.

Ainsi un appareil utilisé à une pression amont de 3,5 bar, peut être utilisé, grâce à l'invention, à une pression de plus de 4 bar, en particulier de d'au moins 4,5 bar, 5 bar ou 5,5 bar, et ce, sans variation significative de sa pression de détente, et le débit de fluide, s'affichant sur le débitmètre situé en aval, n'a pas ses repères de débits faussés

De plus, en cas de modifications de la valeur nominale de la pression de distribution amont aux débitmètres, seul l'élément de raccordement conforme à l'invention devra être changé, dans l'hypothèse où le débitmètre n'en est pas encore équipé, le reste du débitmètre pouvant être conservé. Il en résulte une économie substantielle réalisée lors du passage d'une norme ou d'une réglementation à une autre.

Selon le cas, l'élément de raccordement de l'invention peut comprendre l'une ou plusieurs des caractéristiques techniques suivantes :
- le raccord d'entrée, le raccord de sortie et les moyens de réduction de pression sont disposés coaxialement à la direction d'écoulement du fluide dans le passage interne de l'élément de raccordement.
- le piston est muni d'un clapet dont une extrémité présente des ouvertures de passage de fluide communiquant, d'une part avec le raccord d'entrée, et, d'autre part, avec un conduit traversant ledit clapet et ledit piston.
- ledit moyen de rappel élastique est un ressort.
- le raccord d'entrée et le raccord de sortie sont aménagés aux extrémités du corps et comprennent des moyens de connexions permettant de raccorder l'élément à, d'une part, une prise de distribution de fluide basse pression et, d'autre part, à un débitmètre.
- il comporte une chambre à pression atmosphérique communiquant avec l'atmosphère ambiante via au moins un orifice de communication avec l'extérieur.
- il comporte, en outre, une chambre à la pression du fluide, lesdites chambre à la pression du fluide et chambre à pression atmosphérique étant séparées par le piston.

L'invention porte aussi sur un ensemble comprenant un élément de raccordement selon l'invention, et une prise de distribution de fluide basse pression et/ou un débitmètre, ainsi que sur l'utilisation d'un tel élément de raccordement lors d'une opération de distribution de fluide, en particulier d'un gaz médical, tel l'oxygène, l'air médical ou tout autre gaz.

La description qui va suivre en regard du dessin annexé, donné à titre d'exemple non limitatif, fera bien comprendre en quoi consiste l'invention et comment elle peut être réalisée.

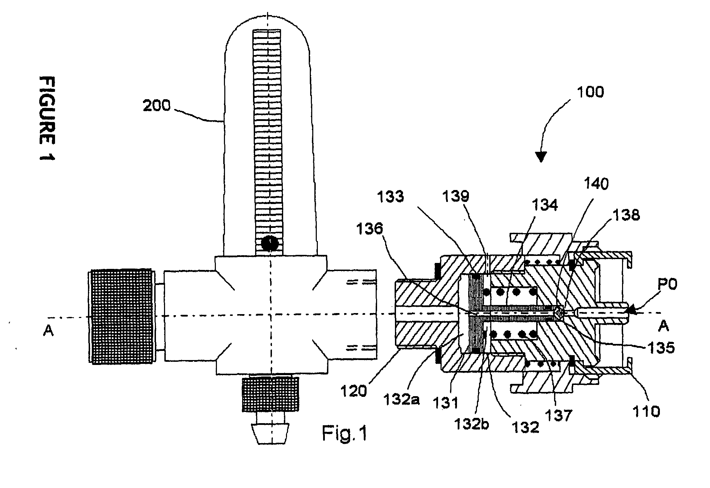

La figure 1 est une vue en coupe d'un élément 100 de raccordement conforme à l'invention destiné à raccorder une prise de distribution de fluide basse pression, non représentée, du type prise murale d'hôpital, à un débitmètre 200 qui peut être un débitmètre à bille apte à fournir le fluide, fluide médical par exemple, sous un débit donné. La pression P₀ du fluide à l'entrée de l'élément 100 de raccordement est typiquement de l'ordre de quelques bars.

L'élément de raccordement 100 comporte un raccord 110 d'entrée de fluide prévu pour raccorder l'élément 100 à la prise de distribution de fluide, ainsi qu'un raccord 120 de sortie de fluide pour raccorder l'élément 100 au débitmètre 200.

Afin de permettre au débitmètre 200 de fonctionner sous pression constante même en cas de variations de la pression amont, l'élément 100 de raccordement comprend des moyens de réduction de la pression du fluide.

Les moyens de réduction de pression sont disposés entre les raccords d'entrée 110 et de sortie 120 coaxialement à la direction d'écoulement du fluide illustrée par l'axe A, lequel constitue un axe de symétrie de révolution pour l'ensemble de l'élément 100 de raccordement.

Ces moyens comprennent un piston 131 apte à se déplacer parallèlement à l'axe A dans un alésage 132, l'étanchéité entre le piston 131 et l'alésage 132 étant réalisée au moyen d'un joint torique 133.

Le piston 131 est muni d'un clapet 134 dont une extrémité présente des ouvertures 135 de passage de fluide, communiquant avec la prise de distribution par l'intermédiaire du raccord 110 d'entrée, ainsi qu'avec un conduit 136 traversant le clapet 134 et le piston 131 de manière à former un passage pour le fluide à travers l'élément 100 de raccordement, depuis la prise de distribution jusqu'au débitmètre 200. Un joint 140 isole la chambre du siège 138 du clapet 134 et la chambre 132b.

Les ouvertures 135 de passage sont pratiquées sur la pente en forme de cône de l'extrémité du clapet 134, laquelle peut venir en appui contre le siège 138.

Par ailleurs, comme visible sur la figure 1, un ressort 137 de rappel, ici un ressort fonctionnant en compression, exerce sur le piston 131 une action combinée avec celle d'une force de pression liée à la pression dans le débitmètre 200, comme expliqué ci-après.

Lorsque l'élément 100 de raccordement muni du débitmètre 200 est connecté à la prise de distribution de fluide, le fluide sous la pression P₀ se répand à l'intérieur de l'ensemble du dispositif en traversant les ouvertures 135 et le conduit 136 de sorte que le piston 131 est soumis, d'une part, sur sa face en regard de la chambre 132a de l'alésage 132, à une force de pression due à l'action exercée sur la surface utile du piston par la pression P₀, qui est alors la pression dans le débitmètre 200, et, d'autre part, à l'action contraire du ressort 137 de compression qui tend à s'opposer à la force pressante de la pression dans le débitmètre 200. La chambre 132b de l'alésage 132 est maintenue à la pression atmosphérique par un orifice 139 de communication avec l'extérieur, percé dans le corps de l'alésage 132.

En l'absence de débit, la force pressante de la pression P₀ repousse le piston 131 de manière à amener l'extrémité du clapet 134 contre le siège 138, ceci du fait que la raideur du ressort 137 est choisie suffisamment faible pour qu'il ne puisse compenser complètement cette force pressante.

Lorsqu'un débit de fluide est demandé à partir du débitmètre 200, la pression dans la chambre 132a diminue, ce qui a pour effet de diminuer la force pressante sur le piston 131 et donc de relâcher le ressort 137 qui peut alors déplacer le piston 131 en sens contraire et dégager l'extrémité du clapet 134 de son siège. Il s'établit alors un régime d'équilibre dans lequel la pression dans la chambre 132a, et donc dans le débitmètre 200, atteint une pression constante, même en cas de variations de la pression amont, dépendant de la valeur de la raideur du ressort 137.

Comme expliqué ci-avant, grâce à la présence combinée de ce piston et du moyen de rappel élastique, on peut garantir une pression de détente stable quelle que soit la variation de pression en amont, en particulier pour une pression de plus de 4 bar.

L'invention trouve une application avantageuse au domaine médical, que ce soit à l'hôpital, dans un service d'urgence, ou pour des soins à domicile, lorsqu'il faut pouvoir fournir à un patient un fluide médical, oxygène ou air principalement, avec la meilleure précision possible quant au débit de fluide fourni.

## Revendications

1. Elément (100) de raccordement destiné à raccorder une prise de distribution de fluide basse pression, en particulier de gaz médical, à un débitmètre (200) apte à fournir ledit fluide sous un débit donné, comprenant un corps principal comportant :
- un raccord (110) d'entrée de fluide apte à raccorder l'élément (100) de raccordement à ladite prise de distribution,
- un raccord (120) de sortie de fluide apte à raccorder l'élément (100) de raccordement audit débitmètre (200),
- un passage interne de fluide reliant le raccord (110) d'entrée au raccord (120) de sortie de fluide, et
- des moyens (131,134) de réduction de la pression du fluide agencés sur ledit passage interne,
**caractérisé en ce que** lesdits moyens (131,134) de réduction de pression comprennent, placés entre le raccord (110) d'entrée et le raccord (120) de sortie, un piston (131) soumis à l'action combinée d'une force de pression liée à la pression dans le débitmètre (200) et à une force de rappel exercée par un moyen (137) de rappel élastique.

2. Elément selon la revendication 1, **caractérisé en ce que** le raccord (110) d'entrée, le raccord (120) de sortie et les moyens (131,134) de réduction de pression sont disposés coaxialement à la direction (A) d'écoulement du fluide dans le passage interne de l'élément (100) de raccordement.

3. Elément selon l'une des revendications 1 ou 2, **caractérisé en ce que** le piston (131) est muni d'un clapet (134) dont une extrémité présente des ouvertures (135) de passage de fluide communiquant, d'une part avec le raccord (110) d'entrée, et, d'autre part, avec un conduit (136) traversant ledit clapet (134) et ledit piston (131), un joint (140) isolant la chambre du siège (138) du clapet (134) et une chambre (132b) à pression atmosphérique.

4. Elément selon la revendication 3, **caractérisé en ce que** ledit moyen de rappel élastique est un ressort (137).

5. Elément selon l'une des revendications 1 à 4, **caractérisé en ce que** le raccord (110) d'entrée et le raccord (120) de sortie sont aménagés aux extrémités du corps principal et comprennent des moyens de connexions permettant de raccorder l'élément (100) à, d'une part, une prise de distribution de fluide basse pression et, d'autre part, à un débitmètre (200).

6. Elément selon l'une des revendications 1 à 5, **caractérisé en ce qu'**il comporte une chambre (132b) à pression atmosphérique communiquant avec l'atmosphère ambiante via au moins un orifice (139) de communication avec l'extérieur.

7. Elément selon l'une des revendications 1 à 6, **caractérisé en ce qu'**il comporte, en outre, une chambre (132a) à la pression du fluide, lesdites chambre (132a) à la pression du fluide et chambre (132b) à pression atmosphérique étant séparées par le piston (131).

8. Ensemble comprenant un élément (100) de raccordement selon l'une des revendications 1 à 7, et une prise de distribution de fluide basse pression et/ou un débitmètre (200).

9. Utilisation d'un élément (100) de raccordement selon l'une des revendications 1 à 7 ou de l'ensemble de la revendication 8, lors d'une opération de distribution de fluide, en particulier d'un gaz médical.
